(19) Europäisches Patentamt

European Patent Office

Office européen des brevets

(11) Publication number: **0 506 965 B1**

# EUROPEAN PATENT SPECIFICATION

(45) Date of publication of patent specification: **28.06.95**   (51) Int. Cl.⁶: **A61K 31/195**, A61K 7/06

(21) Application number: **91912371.1**

(22) Date of filing: **12.07.91**

(86) International application number:
**PCT/JP91/00937**

(87) International publication number:
**WO 92/00733 (23.01.92 92/03)**

(54) **COLLAGEN METABOLISM AMELIORANT AND ITS USE.**

(30) Priority: **13.07.90 JP 186762/90**
         **13.07.90 JP 186763/90**

(43) Date of publication of application:
**07.10.92 Bulletin 92/41**

(45) Publication of the grant of the patent:
**28.06.95 Bulletin 95/26**

(84) Designated Contracting States:
**CH DE ES FR GB IT LI NL**

(56) References cited:
**JP-A-48 005 914**

**No further relevant documents have been disclosed.**

(73) Proprietor: **KANEBO LTD.**
**17-4, Sumida 5 chome**
**Sumida-ku, Tokyo 131 (JP)**

(72) Inventor: **NAKATA, Masanori**
**Seijo Mansion A-302,**
**21-2, Hayakawa 3-chome**
Odawara-shi,
**Kanagawa 250 (JP)**
Inventor: **INOUE, Shintaro**
**20-1, Kotobuki-cho 4-chome,**
**Odawara-shi**
**Kanagawa 250 (JP)**
Inventor: **SOTOMURA, Mikio**
**904-18, Chiyo**
**Odawara-shi,**
**Kanagawa 250-02 (JP)**
Inventor: **TAIRA, Junsei**
**Kopo Yacho 101,**
**160-10, Chimura**
**Hadano-shi,**
**Kanagawa 259-13 (JP)**
Inventor: **MIYAMOTO, Itaru**
**16-2, Higashikaigan Kita 4-chome**
**Chigasaki-shi,**
**Kanagawa 253 (JP)**

(74) Representative: **Hansen, Bernd, Dr.rer.nat. et al
Hoffmann, Eitle & Partner
Patentanwälte
Postfach 81 04 20
D-81904 München (DE)**

## Description

Technical field

The present invention relates to a collagen metabolism ameliorant containing N-methyl-L-serine as an active ingredient, more particularly, a collagen metabolism ameliorant efficacious against collagen hypometabolism caused by aging or diseases accompanied with an abnormal accumulation of collagen, such as hepatic and pulmonary fibroses, keloid, hypertrophic scar, scleroderma, fibrosis in the scalp, or the like.

Furthermore, this invention relates to hair growth stimulants and skin aging inhibitors containing such a collagen metabolism ameliorant.

The term "collagen metabolism amelioration" herein referred to is meant by normalization of a condition such that synthesis of collagen has abnormally progressed due to the loss of balance between synthesis and decomposition of collagen, or reactivation of such a condition as collagen hypometabolism. Further, the term "collagen metabolism" is meant by a phenomenon such that collagen is being renewed at a constant rate as the collagen is undergoing synthesis and decomposition, namely, a metabolic turnover.

Background art

It has so far been suggested that in diseases accompanied with an abnormal accumulation of collagen (hepatic and pulmonary fibroses, keloid, hypertrophic scar, scleroderma, fibrosis in the scalp, or the like), synthesis and decomposition of collagen have been unbalanced [SAISHIN-IGAKU, Vol. 42, No. 10, p.p. 2075-2139 (1987)].

For example, hepatic fibrosis accompanied with hepatic cirrhosis arises from an increase of collagen biosynthesis [Science, Vol. 176, p. 795, (1972)] or a decrease of collagenolytic activity [Biochemical Journal, Vol. 118, p. 229 (1970) and Life Sciences, Vol. 30, p. 1379, (1982)].

Alternatively, it has been known that in the scalp of androgenic alopecia, synthesis and decomposition of collagen are unbalanced, resulting in an abnormal accumulation of collagen, whereby fibrosis of the scalp is promoted [Hair Research, p. 244 (1981), edited by Orfanos, Montagna, Stuttgen, Springer-Verlag Berlin Heidelberg]. In consequence, it is assumed that tensing and a decrease of blood circulation of the scalp, deactivation of hair matrix cells or the like occurs to induce alopecia.

The decrease of the collagenolytic activity is accounted to be caused by a decreased collagenase activity in each organ and/or skin fibroblasts [SKIN, Vol. 14, p. 217 (1972); Journal of Clinical Investigation, Vol. 56, p. 1175 (1975); and Life Sciences, Vol. 30, p. 23 (1982)], so that an enhancement of a collagenase activity is desired. This has been indicated in medical treatments of, for example, hepatic cirrhosis [IGAKU NO AYUMI, Vol. 136, No. 13, p. 1027 (1986)].

Alternatively, metabolism of skin collagen is decreased not only by diseases as mentioned above but also by aging, whereby the collagen remains in the body for a long time and is subjected to a chemical modification to promote crosslinking thereof. It is known that there is formed a vicious circle such that the collagen thus loses its function as an anchorage for cells with the consequence that collagen metabolism is further decreased. Accordingly, it is said that aging of skin may be prevented by restraining the decrease in the collagen metabolism as well as preventing the formation or crosslinkages in collagen [CHEMISTRY TODAY, December, p.36 (1990)].

In order to sever such a vicious circle of the decrease in the collagen metabolism, it is required for collagen not to stay long in the body. For this purpose, an enhancement of the collagenase activity is strongly desired.

The collagenase is a rate limiting enzyme acting during decomposing interstitial collagens (type-I, type-II and type-III collagens) in connective tissues, which plays an important role in collagen metabolism.

The collagenase is produced as a precursor, i.e., procollagenase that is secreted from cells and assumed to be activated in vivo by a protease such as plasmin, stromelysin or the like [Biochemical Journal, Vol. 166, p. 21 (1977) and Proceedings of the National Academy of Sciences of the U.S.A., Vol. 86, p. 2632 (1989)].

From the above, a substance to promote the production of the procollagenase, namely, a collagen metabolism ameliorant, is considered to be efficacious, in medical treatment, against diseases accompanied with an abnormal accumulation of collagen or, in amelioration of collagen metabolism, against collagen hypometabolism caused by aging.

Disclosure of invention

Accordingly, an object of the present invention is to provide a collagen metabolism ameliorant, that is, a collagen metabolism ameliorating agent efficacious against collagen hypometabolism caused by aging or diseases accompanied with an abnormal accumulation of collagen, such as hepatic and pulmonary fibroses, keloid, hypertrophic scar, scleroderma, or fibrosis in the scalp, and further to provide useful hair growth stimulants and skin aging inhibitors.

The present inventors, as a result of assiduous studies for achieving the above-described object, have found that N-methyl-L-serine has an action of promoting the production of the procollagenase, and accomplished the present invention.

Namely, the present invention relates to a collagen metabolism ameliorant containing N-methyl-L-serine as an active ingredient and its use.

The collagen metabolism ameliorant according to the present invention can be compounded with a known ingredient according to its use object and prepared into a dosage form suited for peroral administration or parenteral administration such as injection, percutaneousness or the like, by a usual process. This ameliorant is administered perorally or parenterally, such as by injection, percutaneousness or the like, to the human body.

As a dosage form for peroral administration, mention may be made of solid preparations such as tablets, granules, powders, particulates, hard gelatine capsules and the like, and in addition, solutions such as syrups, elixirs, elastic capsules and the like.

Injections are prepared by dissolving or emulsifying N-methyl-L-serine in physiological salt solution or a lipid diluting agent such as vegetable oils, oil emulsions, glycols or the like, and encapsulating germfreely into ampuls or vials.

Percutaneous medicaments include ointments, lotions, cataplasms, gels, creams, solutions, aerosols, baths, patches and the like.

The collagen metabolism ameliorant according to the present invention is administered perorally or parenterally. In the case of a disease accompanied with an abnormal accumulation of collagen in an organ, such as hepatic fibrosis, pulmonary fibrosis and the like, the collagen metabolism ameliorant of the present invention is administered perorally or by injection, and in the case of a disease accompanied with an abnormal accumulation of collagen in skin, such as keloid, hypertrophic scar, fibrosis in the scalp, and the like, it is administered percutaneously or by a regional injection.

Alternatively, in the case of collagen hypometabolism caused by aging, the collagen metabolism ameliorant according to the present invention is administered perorally or percutaneously.

Though the dosage depends upon the age, weight, conditions of a patient to be administered, dosage form and the like, in the case of adults, generally a dose of 0.1-1,000 mg as N-methyl-L-serine and 1-3 doses a day are administered.

For example, in the case of a disease accompanied with an abnormal accumulation of collagen in an organ, such as hepatic fibrosis, pulmonary fibrosis and the like, a dose of 30-1,000 mg by peroral or injection administration is appropriate, while in the case of a disease accompanied with an abnormal accumulation of collagen in skin, such as keloid, hypertrophic scar, fibrosis of the scalp and the like, or a skin with collagen hypometabolism caused by aging, a dose of 0.1-50 mg by percutaneous administration is appropriate.

Alternatively, the collagen metabolism ameliorant according to the present invention can be used as a hair growth stimulant and a skin aging inhibitor.

The hair growth stimulant can be compounded with a known material according to a usual process and prepared into preventives for hair falling-off, hair-restorers, and further into the form of a hair-restoring cosmetics such as shampoo, rinse, hair tonic, hair lotion, hair cream, hair conditioners, hair gel, hair mist, hair foam and the like.

In the hair growth stimulant, N-methyl-L-serine is compounded in an amount of 0.001-10.0% by weight (hereinafter abbreviated as "wt.%") (based on the total amount), preferably 0.01-5.0 wt.%.

The skin aging inhibitor can be compounded with a known material according to a usual process and prepared into dermal cosmetics such as wrinkle preventives, ointments and, further into the form of skin lotion, skin milk, skin cream, pack or the like.

In the skin aging inhibitor, N-methyl-L-serine is compounded in an amount of 0.001-10.0% by weight (hereinafter abbreviated as "wt.%") (based on the total amount), preferably 0.01-5.0 wt.%.

Additionally, as the collagen metabolism ameliorant according to the present invention, though N-methyl-L-serine is mentioned as the above, pharmacologically allowable salts thereof also can be likewise utilized.

4

Best mode for carrying out the invention

The present invention will be explained in more detail hereinafter by way of experimental examples, examples and comparative examples.

In the outset, assessments of N-methyl-L-serine, etc. are described with respect to pharmacological, experimental examples, and then assessments regarding the after-described examples and comparative examples are described.

[Experimental Example-1] Action of promoting procollagenase production.

1) Abbreviations.

The following abbreviations used in the experimental examples have the under-noted meanings.

HF medium: a medium prepared by dissolving 10.6 g of Ham's F12 powdery medium (manufactured by Nissui Pharmaceutical Co.) in refined water.

HF-AV medium: a medium prepared by admixing 1 liter of the HF medium with 1.76 g of a powdery Eagle's aminoacid vitamin medium (manufactured by Nissui Pharmaceutical Co.), 1.6 g of sodium carbonate, 50 mg of streptomycin sulfate and 60 mg of kanamycin sulfate and then blowing carbonic acid gas thereinto to adjust the pH value to about 7.

TRIS: tris(hydroxymehtyl)aminomethane.

MES buffer solution: a buffer solution prepared by adjusting the pH value of a 50mM 2-(N-morpholino)-ethane sulfonic acid monohydrate aqueous solution containing a 0.5M NaCl, a 1mM $CaCl_2$ and 0.05% by volume of BRIJ-35 (the trademark of polyoxyethylene laurylalcohol) to 6.5 at 4°C with a TRIS aqueous solution.

Acetic acid buffer solution: a buffer solution prepared by adjusting the pH value of a 25mM acetic acid aqueous solution containing a 0.5M NaCl, a 1mM $CaCl_2$ and 0.05% by volume of BRIJ-35 to 4.5 at 4°C with a TRIS aqueous solution.

MES-A buffer solution: a buffer solution prepared by adjusting the pH value of the MES buffer solution to 7 at 4°C with a TRIS aqueous solution.

MES-B buffer solution: a buffer solution prepared by mixing the MES buffer solution with the acetic acid buffer solution and adjusting the pH value to 2 at 4°C.

Buffer solution for measurement: a buffer solution prepared by adjusting the pH value of a 50mM TRIS aqueous solution containing a 0.2M NaCl, a 5mM $CaCl_2$ and 0.05% by volume of BRIJ-35 to 7.5 at room temperature with hydrochloric acid.

2) Test compound.

N-methyl-L-serine (hereinafter abbreviated as "MSE").

3) Cell employed.

A test was carried out using anchorage independent cell strain which is derived from a human fibrosarcoma cell strain HT 1080 (ATCC CCL121) and growable in a serum-free, protein-free medium (referred to as "human fibrosarcoma cell strain HT-P11". The cells were pre-cultured as below to prepare a cell suspension which was then tested.

The human fibrosarcoma cell strain HT-P11 was suspended with a density of $1 \times 10^5$ cells/ml in the HF-AV medium. Flasks (each having a base area of 75 cm$^2$) were charged, respectively, with 20 ml or the resulting suspension, and stationarily cultured at 37°C for 3 days in a 95% air/5% carbonic acid gas atmosphere.

After culturing for 3 days, the cells were collected by centrifugation (at 600 rpm for 10 min.). The obtained cells were suspended in the HF-AV medium to prepare a cell suspension with a density of $7 \times 10^4$ cells/ml.

4) Test method.

Two milliliters each of the above cell suspension was laid on a 6 well plate (with a base area of 9.4 cm$^2$) and cultured at 37°C for 1 day in a 95% air/5% cabonic acid gas atmosphere. Then, a 10 mM conc. test compound aqueous solution was diluted into a 600 $\mu$M concentration with the HF-AV medium. After sterilizing by filtering with a nitrocellulose membrane having a pore size of 0.2 $\mu$m (the trademark "DISMIC-25", manufactured by Advantec Toyo Co.), 0.4 ml each of the above solution was added to culture solutions and cultured for 13 days in a 95% air/5% carbonic acid gas atmosphere. After completing culturing, 1 part by volume of a 10 vol.% BRIJ-35 aqueous solution was added to 200 parts by volume of the culture solution. Then, the cells were separated by centrifugation (at 600 rpm for 10 min.) and a culture supernatant liquor was obtained.

Then, 0.5 ml of the culture supernatant liquor was admixed with 0.5 ml of the MES-A buffer solution and the mixture was charged into a column loaded with 0.5 ml of zinc chelating SEPHAROSE 6B™ -(manufactured by Pharmacia) equilibrated by the MES-A buffer solution. By 4 repetitive flows of 1 ml each of the MES-B buffer solution through the column, collagenase inhibitors were eluted from the column and removed.

Then, by 2 repetitive flows of 1 ml each of the MES-C buffer solution, eluting solution was collected and 2 ml of procollagenase were obtained.

After adjusting the pH value of the procollagenase solution to about 7 with a 1N NaOH, the volume of the solution was increased to 2.5 ml by adding the MES-A buffer solution thereto. Then, using the buffer solution for measurement, a solution containing about 0.1-0.7 unit/ml of the procollagenase was prepared as a test liquid.

Then, 50 $\mu$l of the test liquid were admixed with 20 $\mu$l of a trypsin solution ("Type 12" manufactured by Sigma Chemical, adjusted into a 1 mg/ml concentration with the buffer solution for measurement) and, after incubating at 35°C for 5 min., further admixed with 30 $\mu$l of a soybean trypsin inhibitor solution ("No. 24020" manufactured by Merck, was adjusted into a 3 mg/ml concentration with the buffer solution for measurement) to inactivate the trypsin and a collagenase solution was obtained.

Using a type-I collagen labeled with fluorescein isothiocyanate (hereinafter abbreviated as "FITC") (a 1 mg/ml conc. FITC/collagen acetic acid solution, manufactured by Cosmo Bio Co.:) as a substrate solution, the activity (unit/ml) of the above collagenase solution was determined in accordance with the Nagai et al's Method [Inflammation, Vol.4, No. 2, p. 123 (1984)]. With respect to the collagenase produced from the procollagenase by the above-mentioned trypsin treatment, an amount enough to decompose 1 $\mu$g/min. of the type-I collagen (FITC/collagen) at 35°C, is assumed to be a 1 unit procollagenase and the production of the procollagenase (unit/ml of culture solution) was found (let this value be X).

On the other hand, as a control experiment, refined water in lieu of the test compound aqueous solution was added and the production of the procollagenase (unit/ml of culture solution) in the absence of the test compound was found by the same procedure as the above (let this value be Y).

Then, from these values, a procollagenase production promotion rate (%) was calculated by the following equation

$$\text{Procollagenase production promotion rate } (\%) = \frac{X - Y}{Y} \times 100$$

5) Test result

The results are shown in Table 1.

Table 1

| Test compound | Production of procollagenase (unit/ml of culture solution) | Production promotion rate (%) |
|---|---|---|
| No additives (control) | 3.7 ± 0.2 | - |
| MSE | 7.9 ± 0.7 ** | 114 |
| The data shows a mean value ± standard error (n = 3). | | |

** As P<0.01, significantly different as compared with no additives (control) (by Duncan's method).

As is clear from Table 1, N-methyl-L-serine (MSE) promoted the production of the procollagenase.

[Experimental Example-2] Effect in medical treatment for hepatic fibosis.

A model animal of hepatic fibosis was prepared and tested.

1) Test compound.

MSE

2) Test method.

In accordance with a drug action screening method of an effect for amelioration of a liver function (H.OZAWA: "Pharmacological Screening Methods for New Drug Development", p. 75, (1984), published by MARUZEN), a 2 ml/kg dose of an equivalent mixture or carbon tetrachloride (hereinafter abbreviated as "CCl$_4$") and olive oil was administered hypodermically to the back of male rats of Wistar origin (5 weeks old; a weight of 112-134 g; and 5 rats/group) twice a week (on Monday and Thursday) for 10 weeks to produce rats of hepatic fibosis. A 3 ml/kg dose of a 66.6 mg/ml conc. test compound aqueous solution was administered perorally once a day for continual 7 weeks (except Sundays) from the 4th week after the commencement of the CCl$_4$ administration.

Then, the whole blood was gathered from the abdominal aorta and collected in a test tube treated with heparin. This was centrifuged to obtain blood plasma. The blood plasma was stored at -80°C until the under-mentioned biochemical examinations.

Further, after gathering the whole-blood, the liver was extracted, weighed and divided into halves under an ice-chilled condition. Then, one of the halves was frozen at -80°C for quantification of hydroxyproline (hereinafter abbreviated as "Hyp") in the liver and the other half was fixed with a 10% formaline solution for a histological examination.

On the other hand, there were provided, as an untreated group, a group of the rats of hepatic fibosis administered with 3 ml/kg of refined water in lieu of the test compound aqueous solution according to the same schedule as the above and, as a group of normal rats, a group bred for 10 weeks without administering carbon tetrachloride and the test compound aqueous solution. Blood-gathering and liver-extraction were conducted on these groups in the same manner as the above.

Then, using the above each liver and blood plasma, the Hyp in the liver and blood were quantified. Additionally, the Hyp is an index of the quantity of the collagen [Methods of Biochemical Analysis, Vol. 15, p. 25 (1967)]. Further, biochemical examinations of the blood plasma [refer to the under-described items (b)-(h)] and histological examinations of the liver [refer to the under-described item (i)], as indices of liver injury, were conducted.

Each examination item and examination method are as follows.
(a) Quantities of the Hyp in liver and blood.
(a-1) Quantity of the Hyp in a liver: about 100 mg of a liver were weighed precisely and added with 250 $\mu$l or physiological salt solution. Then, the liver was disintegrated by vigorously agitating (at 28,000 rpm for 30 seconds) with PHYSCOTRON™ (a homgenizer manufactured by Niti-On Medical and Physical Instruments Mfg. Co.).

The obtained suspension was centrifuged (at 15,000 rpm for 10 min.) and separated into a supernatant sample and a precipitation sample. By adding a 6N hydrochloric acid, each sample was increased to 2 ml and hydrolyzed at 110°C for 24 hours. The hydrolyzate solution was centrifugally condensed to remove hydrochloric acid therefrom and the condensate was dissolved in a 0.01N hydrochloric acid.

The obtained solution was quantified according to a determination method of amino acids by a high-performance liquid chromatography with a post-column derivatization [Proceedings of the National Academy of Sciences of the U.S.A., Vol. 72, No. 2, p. 619 (1975)] to find quantities of the Hyp in the supernatant sample and precipitation sample, respectively. Then, a quantity of the Hyp per unit weight of the liver ($\mu$mol/g of liver) was calculated from the total quantity of the Hyps in the supernatant sample and precipitaion sample and the weight of the liver.
(a-2) Quantity of the Hyp in blood: 100 $\mu$l of blood plasma was admixed with 100 $\mu$l of a 5 w/v % sulfosalicylic acid aqueous solution under an ice-chilled condition and centrifugally separated (10,000 rpm; for 3 min.) to obtain a supernatant liquor. The Hyp in the supernatant liquor was quantified according to the abovementioned determination method of amino acids ($\mu$mol/l).
(b) Quantity of glutamic-pyruvic transaminase (hereinafter abbreviated as "GPT"): this was determined according to the Karmen's method [Izumi KANAI: RINSYO KENSA HOU TEIYO (Manual of Clinical Examination Method), Revised Edition No.29, p.514 (1983), published by Kimbara Publishing Co.] (unit/l).

(c) Quantity of glutamic-oxaloacetic transaminase (hereinafter abbreviated as "GOT"): this was determined according to the Karmen's method [Izumi KANAI: RINSYO KENSA HOU TEIYO, Revised Edition No.29, p.514 (1983), published by Kimbara Publishing Co.] (unit/l).

(d) Quantity of γ-glutamyl transpeptidase (hereinafter abbreviated as "γ-GTP"): this was determined according to a method using L-γ-glutamyl-p-nitroanilide as a substrate [Izumi KANAI: RINSYO KENSA HOU TEIYO, Revised Edition No.29, p.532 (1983), published by Kimbara Publishing Co.] (unit/l).

(e) Quantity of gross bilirubin: determined according to the Malloy-Evelyn's method [Izumi KANAI: RINSYO KENSA HOU TEIYO, Revised Edition No.29, p.724 (1983), published by Kimbara Publishing Co.] (mg/dl).

(f) Quantity of alkaline phosphatase: this was determined according to the Bessey-Lowry's method [Izumi KANAI: RINSYO KENSA HOU TEIYO, Revised Edition No.29, p.496 (1983), published by Kimbara Publishing Co.] (unit/l).

(g) Thymol turbidimetric test value (hereinafter abbreviated as "TTT"): this was determined according to the standard method of Committee for Liver Function Test, the Japanese Society of Gastroenterology [Izumi KANAI: RINSYO KENSA HOU TEIYO, Revised Edition No.29, p.711 (1983), published by Kimbara Publishing Co.] (U).

(h) Zinc sulphate turbidimetric test value (hereinafter abbreviated as "ZTT"): this was determined according to the standard method of Committee for Liver Function Test, the Japanese Society of Gastroenterology [Izumi KANA: RINSYO KENSA HOU TEIYO, Revised Edition No. 9, p.710 (1983), published by Kimbara Publishing Co.] (U).

(i) Histological examination of liver: a liver was sliced according to a usual method and, after hematoxylin-eosin staining, it was microscopically observed.

3) Test result.

The quantities of the Hyp in the liver and blood as well as biochemical examination values of the blood plasma, as an index of liver injury, are shown in Table 2.

Table 2

| Exam item | Group of normal rats | Rats of hepatic fibrosis induced by carbon tetrachloride | |
| --- | --- | --- | --- |
| | | Untreated group | MSE administered group |
| a-1 | 1.72±0.63 | 10.0±1.5 | 6.79±0.82* |
| a-2 | 27.6±2.3 | 30.6±1.1 | 39.1±3.6* |
| b | 45.2±3.8 | 948±92 | 537±182** |
| c | 85±6.8 | 2170±184 | 875±324** |
| d | 1.8±0.6 | 17.6±0.1 | 14.4±1.7* |
| e | <0.10 | 0.36±0.02 | 0.26±0.05* |
| f | 317±13 | 1270±90 | 956±114** |
| g | 0.10±0.03 | 0.26±0.06 | 0.18±0.13 |
| h | 0.28±0.08 | 0.78±0.11 | 0.26±0.07** |
| The data shows a mean value±standard error (n = 5). | | | |

* As $P<0.05$, significantly different as compared with the untreated group (by Duncan's method).
** As $P<0.01$, significantly different as compared with the untreated group (by Duncan's method).

As shown in Table 2, the MSE administered group had significantly a low Hyp content in the liver and significantly a high Hyp content in blood, as compared with the untreated group. This shows that the collagen metabolism ameliorant of the present invention promotes the production of the procollagenase with the consequence that the decomposition of the collagen in the fibrotic hepatic tissue is promoted, resulting in isolation of the Hyp in blood.

Further, the biochemical examination values of the blood plasma of the MSE administered group are significantly low with respect of GPT, GOT, γ-GTP, gross bilirubin, alkaline phosphatase and ZTT, as compared with those of the untreated group. From this fact, it can be said that the collagen metabolism ameliorant of the present invention ameliorated a liver injury accompanied with hepatic fibrosis.

8

On the other hand, in a histological examination of the liver, it was observed that the MSE administered group mitigated a liver-injury-based, diffuse hepatocyte vacuolization, cholangiolar hyperplasia and cholangioepithelial cytogenesis, respectively, as compared with the untreated group. This fact also shows that the collagen metabolism ameliorant of the present invention is efficacious against a liver injury accompanied with hepatic fibrosis.

[Experimental Example-3] Skin viscoelasticity test.

1) Test compound.

A pharmacopoeial water-absorbing ointment was blended with the compounding materials of the under-described examples or comparative example in the under-described contents (weight %, hereinafter abbreviated as "wt.%") and test creams were prepared (additionally, an ointment without compounding these materials was used as a control).

2) Test method.

Male hairless rats of Wistar strain (6 weeks old; a weight of 110g-130 g; 5 rats/group) were hair-trimmed at their dorsal region. A test compound was applied everyday in an amount of 0.1 g/2 cm $\times$ 2 cm area onto a right shoulder position. On the 45th day after the commencement of the test, a skin-viscoelasticity was measured with a vibration-type skin-viscoelastometer described in Japanese Patent Laid-open Application No. 59-120,130.

The mean viscoelasticity (S) of the group applied with the test compound blended cream and the mean viscoelasticity (C) of the group applied with a cream without blending the test compound therewith were found. Then, (S)/(C) was calculated to assess the viscoelasticity of the skin.

Additionally, as to the skin-viscoelasticity displayed on the above skin-viscoelastometer (in an arbitrary unit), the higher the value, the softer the skin is.

3) Test result.

The results are shown in Tables 3-6.

As is clear from those tables, all the samples of the example showed a promotion effect on skin-viscoelasticity.

[Experimental Example-4] Human use test of hair growth promotion effect.

1) Test compound.

The samples of the after-described examples or comparative examples were used.

2) Test method.

Ten testees who were patients of androgenic alopecia had the hair of the head shaved into a round shape of a 1 cm diameter at two testing positions 5 cm above the both ears. The testing position on the left side was applied with about 3 ml of a test compound every morning and every evening and compared with the untreated, right side position. On the 28th day from the commencement of the test, 20 hairs each on both the testing positions were shaved off. The mean length (B) on the left side (applied with the compound of the example or comparative example) and the mean length (A) on the right side (untreated) were found. The appraisal of the effect was shown by the mean (B)/(A) of 10 testees.

3) Test result.

The results are shown in Tables 3 and 4.

As is clear from the tables, all the samples of the example exhibited a growth promotion effect.

[Experimental Example-5] Skin blood flow promotion test.

1) Test compound.

The samples of the after-described example or comparative example were used.

2) Test method.

Three female rabbits of New Zealand white strain were hair-trimmed in an abdominal region. After fasting for 18 hours, 35 mg/kg of a pentobarbital sodium salt were intravenously injected to anesthetize. Immobilizing the back of the rabbit, 0.1 g each of the test compound and a non-blended compound was applied uniformly onto two zones of 3 cm × 2 cm in the abdominal region. On these zones, a plate-type transducer was fixed with a cellophane tape, and with a crossed thermocouple type skin blood flowmeter (the trademark "SHINCORDER" Model 201, manufactured by Shin-Ei Co.), a skin blood flow ($\mu$V) was measured after 0.1, 1.0 and 2.0 hours from applying of the test samples.

Let the quantity of the blood flow when the test compound was applied be (A) and the quantity of the blood flow in the case of the non-blended compound be (B), and a blood flow ratio (A)/(B) was calculated. The mean value was found from all values obtained from every measurement at the above time with respect to three rabbits. Then, the mean values of the blood flow ratio at respective times were further averaged and thus averaged value represented a skin-blood flow increase ratio.

3) Test result.

The results are shown in Tables 5 and 6.
As is clear from the tables, the samples of the example exhibited a blood flow promotion effect.

Table 3

| Sample | Compounded material | Content (wt%) | Skin-viscoelasticity improvement | Human hair growth promotion |
|---|---|---|---|---|
| Example 1 | MSE | 0.1 | 1.11 | 1.18 |
| Example 2 | MSE | 0.5 | 1.30 | 1.25 |
| Example 3 | MSE | 4.0 | 1.42 | 1.34 |
| Comparative Example 1 | Red pepper tinctures | 0.1 | 0.95 | 1.01 |
| Comparative Example 2 | Japanese swertia extracts | 5.0 | 1.02 | 1.02 |
| Comparative Example 3 | Ethyl nicotinate | 0.1 | 1.01 | 1.00 |

Table 4

| Sample | Compounded material | Content (wt%) | Skin-viscoelasticity improvement | Human hair growth promotion |
|---|---|---|---|---|
| Example 4 | MSE | 0.2 | 1.15 | 1.15 |
| Example 5 | MSE | 3.0 | 1.36 | 1.38 |
| Comparative Example 4 | Red pepper tinctures | 0.1 | 1.00 | 1.01 |
| Comparative Example 5 | Biotin | 0.1 | 1.02 | 1.00 |
| Comparative Example 6 | Panthenol | 2.0 | 0.98 | 1.02 |

Table 5

| Sample | Compounded material | Content (wt%) | Skin-viscoelasticity improvement | Blood flow promotion |
|---|---|---|---|---|
| Example 6 | MSE | 0.1 | 1.12 | 1.20 |
| Example 7 | MSE | 1.0 | 1.32 | 1.23 |
| Example 8 | MSE | 4.0 | 1.43 | 1.50 |
| Comparative Example 7 | γ-oryzanol | 5.0 | 1.01 | 1.05 |
| Comparative Example 8 | Ethyl nicotinate | 0.1 | 1.02 | 1.01 |

Table 6

| Sample | Compounded material | Content (wt%) | Skin-viscoelasticity improvement | Blood flow promotion |
|---|---|---|---|---|
| Example 9 | MSE | 0.2 | 1.16 | 1.27 |
| Example 10 | MSE | 4.0 | 1.35 | 1.37 |
| Comparative Example 9 | Panthenol | 5.0 | 0.99 | 1.03 |
| Comparative Example 10 | Biotin | 0.1 | 1.01 | 1.05 |

[Experimental Example-6] Effect on skin collagen.

Hairless rats having an insoluble skin collagen increased with aging were used for evaluation.

1) Test compound.

The ointment prepared in Example 11 (hereinafter abbreviated as "MSE ointment") and the ointment prepared in Comparative Example 11 (hereinafter abbreviated as "control ointment") were used.

2) Test method.

Fifteen male hairless rats of Wistar origin (6 weeks old; a weight of 110-130 g) were divided into 3 groups each consisting of 5 rats. Among them, one group was applied with the control ointment and another one group was applied with the MSE ointment, in an amount of 0.1 g onto the back right shoulder, once a day for continual 60 days. After 60 days, skins were cut off and subcutaneous fat was removed therefrom to provide skins of a 15 weeks old rat group applied with the control ointment (aged skins) and of a 15 weeks old rat group applied with the MSE ointment, respectively.

With respect to the remaining one group, skins were cut off from the back of the rats the instant that the test was commenced, to provide skins of an untreated, 6 weeks old rat group (young skins).

About 200 mg of the cut skin were added with 1 ml of physiological salt solution and subjected to 2 repetitive homogenizations at 28,000 rpm for 30 seconds with a homogenizer (manufactured by Niti-On Medical & Physical Instruments Mfg. Co.). The blades of the homogenizer were washed with 1 ml of physiological salt solution and the saline was added to the above homogenized test sample. Further adding physiological saline, 2.4 ml of suspension per 100 mg of skin were provided.

The obtained suspension was shaken at 4°C at 120 rpm for 24 hours and then centrifuged at 2,000 g for 10 minutes. Thus, a fraction solubilizable at 4°C was obtained.

To the remaining precipitate, 10 ml of a Ringer's solution were added to homogenize. After shaking at 65°C at 120 rpm for 45 minutes, centrifugation was conducted at 2,000 g for 10 minutes to provide a fraction solubilizable at 65°C.

To the remaining precipitate, 5 ml of refined water were added to homogenize and, after autoclaving at 125°C for 60 minutes, centrifugation was conducted at 3,000 rpm for 10 minutes to provide a fraction solubilizable at 125°C.

The volume of these fractions was measured and each 1 ml of the fractions was added with 1 ml of a 12N hydrochloric acid to completely hydrolyze at 110°C for 24 hours. Then, hydrochloric acid was removed by centrifugal condensation and the resultant was redissolved with 1 ml of a 10 mM hydrochloric acid. With regard to the redissolved solution, the Hyp was quantified according to the determination method of amino acids shown in Experimental Example 2. The obtained value represented the quantity of the collagen.

3) Test result.

The results are shown in Table 7.

Table 7

| Treatment | Collagen ($\mu$mol Hyp/g of skin) [1] | | |
| --- | --- | --- | --- |
| | (Solubilizing temperature) | | |
| | 4°C | 65°C | 120°C |
| 6 weeks old rats · Untreated | 42 ± 2 | 81 ± 6 | 11 ± 1 |
| 15 weeks old rats · Control ointment applied | 24 ± 5 | 157 ± 6 | 30 ± 2 |
| · MSE ointment applied | 44 ± 2 | 114 ± 3 | 26 ± 1 |

1) Mean value ± standard error   (n = 5)

From the above table, it has been clarified that the MSE ointment restrains an increase of a constitutional proportion of a hard-extractable collagen (collagen with many crosslinkages) with aging and acts to maintain the constitutional proportion of the collagen on the level of 6 weeks old.

[Experimental Example-7] Acute toxicity.

An acute toxicity was examined as follows:

1) Test compound.

MSE.

2) Test method and result.

To 5 male mice of ICR strain (5 weeks old; a weight of 24-28 g), a 25 g/ml conc. test compound aqueous solution that had been prepared by dissolving the test compound in refined water was perorally administered once in a dose of 0.2 ml/kg of the body weight (5 g/kg of the body weight, as the test compound).

Then, the mice were observed for 7 days and no deaths by administration of the test compound were recognized.

[Experimental Example-8] Skin irritative test.

Using male rabbits of Japanese native strain (a weight of about 3 kg), a skin irritative test was conducted in accordance with the Draize method [Appraisal of the Safety of Chemicals in Foods, Drugs and Cosmetics, p.46 (1959) edited and published by the Editorial Committee, Association of Food & Drug Officials of U.S.A.].

12

1) Test compound.

MSE.

2) Test method.

An abraded region (injured region) was produced on the hair-trimmed back of the rabbits. An adhesive plaster for a patch test (RIBBON-AID™ of 1.2 cm × 1.6 cm, manufactured by Liba-Tape & Co., Ltd.) was impregnated with 0.1 ml of a 1 wt.% test compound aqueous solution that had been prepared by dissolving the test compound in refined water and attached onto the injured skin and a normal skin, respectively. After 24 hours, the plaster was detached to observe erythematic and edematous conditions of the skins. After 48 hours from detaching of the plaster, the same observation was made. Erythematic scores and edematous scores were recorded based on the criteria of assessment shown in Table 8.

Table 8

| Criterion of assessment | Erythematic score | Edematous score |
|---|---|---|
| No erythema nor edema | 0 | 0 |
| Slight erythema or edema | 1 | 1 |
| Mild erythema or edema | 2 | 2 |
| Medium erythema or edema | 3 | 3 |
| Severe erythema or edema | 4 | 4 |

Based on the above scores and the following equation, primary irritant scores were calculated.

$$\text{Primary irritant score} = (A_{24} + A_{48})/2 + (B_{24} + B_{48})/2 + (X_{24} + X_{48})/2 + (Y_{24} + Y_{48})/2$$

where each symbol has the following meaning:

$A_{24}$: an erythematic score after 24 hours from attaching the plaster onto the normal skin,
$A_{48}$: an erythematic score after 48 hours from detaching the plaster from the normal skin,
$B_{24}$: an erythematic score after 24 hours from attaching the plaster onto the injured skin,
$B_{48}$: an erythematic score after 48 hours from detaching the plaster from the injured skin,
$X_{24}$: an edematous score after 24 hours from attaching the plaster onto the normal skin,
$X_{48}$: an edematous score after 48 hours from detaching the plaster from the normal skin,
$Y_{24}$: an edematous score after 24 hours from attaching the plaster onto the injured skin, and
$Y_{48}$: an edematous score after 48 hours from detaching the plaster from the injured skin.

Then, irritation degrees of the test compounds were appraised based upon the primary irritative scores and the criteria shown in Table 9.

Table 9

| Primary irritative score | Appraisal |
|---|---|
| 0 ~ less than 2 | mild irritation |
| 2 ~ less than 5 | moderate irritation |
| 5 or more | severe irritation |

3) Test result.

As the results of the skin irritative test, the primary irritative score was found to be zero which was appraised as light irritation.

13

[Experimental Example-9] Hair-growth practicality test.

1) Test compound.

The same as Experimental Example-4 (the samples of the after-described examples or comparative examples were used).

2) Test method.

Onto the heads of 20 testees who were patients of androgenic alopecia, a test compound was applied every morning and evening for continual 6 months. Then, the effects were appraised. The hair growth stimulative effect, hair falling-off preventive effect, dandruff preventive effect were shown by the number of the testees answered "downs were thickened or increased in number", "hair falling-off was lessened" and "dandruff was decreased", respectively.

3) Test result.

The results are shown in Tables 10 and 11.

Table 10

| Sample | Compounded material | Content (wt%) | Practicality test (No. of persons) | | |
|---|---|---|---|---|---|
| | | | Hair growth | Prevention of hair falling-off | Prevention of dandruff |
| Example 1 | MSE | 0.1 | 15 | 15 | 10 |
| Example 2 | MSE | 0.5 | 16 | 17 | 12 |
| Example 3 | MSE | 4.0 | 18 | 17 | 14 |
| Comparative Example 1 | Red pepper tinctures | 0.1 | 5 | 7 | 4 |
| Comparative Example 2 | Japanese swertia extracts | 5.0 | 4 | 6 | 6 |
| Comparative Example 3 | Ethyl nicotinate | 0.1 | 5 | 6 | 5 |

Table 11

| Sample | Compounded material | Content (wt%) | Practicality test (No. of persons) | | |
|---|---|---|---|---|---|
| | | | Hair growth | Prevention of hair falling-off | Prevention of dandruff |
| Example 4 | MSE | 0.2 | 16 | 15 | 11 |
| Example 5 | MSE | 3.0 | 18 | 18 | 13 |
| Comparative Example 4 | Red pepper tinctures | 0.1 | 4 | 7 | 4 |
| Comparative Example 5 | Panthenol | 2.0 | 5 | 5 | 6 |
| Comparative Example 6 | Biotin | 0.1 | 4 | 5 | 5 |

From these tables, the effect was clearly recognized also in the practicality test.

[Experimental Example-10] Practicality-to-skin test.

1) Test compound.

The same as Experimental Example-5 (the samples of the after-described examples or comparative examples were used).

14

2) Test method.

Twenty female testees (35-55 years of age) who complained of rough skin, fine wrinkles, dry skin and the like, were made to use a test compound every morning and evening for continual 3 months. Then, the testees appraised the effect with respect to improvements of skin in softness, elasticity and wrinkles. The effects of the above items were shown by the number of the testees answered "the softness of skin has been improved", "the elasticity of skin has been improved" and "the wrinkles of skin have been ameliorated", respectively.

3) Test result.

The results are shown in Tables 12 and 13.

Table 12

| Sample | Compounded material | Content (wt%) | Practicality test (No. of persons) | | |
|---|---|---|---|---|---|
| | | | Softness | Elasticity | Wrinkle amelioration |
| Example 6 | MSE | 0.1 | 14 | 16 | 13 |
| Example 7 | MSE | 1.0 | 17 | 18 | 14 |
| Example 8 | MSE | 4.0 | 16 | 18 | 15 |
| Comparative Example 7 | $\gamma$-oryzanol | 5.0 | 6 | 8 | 8 |
| Comparative Example 8 | Ethyl nicotinate | 0.1 | 7 | 9 | 7 |

Table 13

| Sample | Compounded material | Content (wt%) | Practicality test (No. of persons) | | |
|---|---|---|---|---|---|
| | | | Softness | Elasticity | Wrinkle amelioration |
| Example 9 | MSE | 0.2 | 14 | 16 | 12 |
| Example 10 | MSE | 4.0 | 19 | 19 | 15 |
| Comparative Example 9 | Panthenol | 5.0 | 7 | 6 | 7 |
| Comparative Example 10 | Biotin | 0.1 | 6 | 8 | 7 |

From these tables, the effect was clearly recognized also in the practicality test.
The present invention will be explained hereinafter by way of examples and comparative Examples.

[Example 1] ~ [Example 3]

The MSE was added to and dissolved in the under-described hydrophilic ingredients in an amount of 0.1 wt.% (Example 1), 0.5 wt.% (Example 2) or 4.0 wt.% (Example 3). Then, the resulting solution was mixed with the under-described lipophilic ingredients and stirred to disperse therein. Thus, oily hair tonics were prepared.

| Starting ingredient | Content (wt.%) |
|---|---|
| (Hydrophilic ingredient) | |
| Glycerin<br>Aromatic<br>Refined water | 5.0<br>0.1<br>balance |
| (Lipophilic ingredient) | |
| Olive oil<br>Isopropyl myristate<br>Isopropylmethylphenol<br>Polyoxyethylene nonylphenylether (20 E.O.)<br>Methyl parahydroxybenzoate<br>Ethanol | 5.0<br>2.0<br>0.05<br>0.5<br>0.1<br>60.0 |

[Comparative Example 1] ~ [Comparative Example 3]

Oily hair tonics were prepared in the same manner as Example 1, except that the MSE was replaced by 0.1 wt.% of red pepper tinctures (Comparative Example 1), 5.0 wt.% of Japanese swertia extracts (Comparative Example 2) or 0.1 wt.% of ethyl nicotinate (Comparative Example 3).

[Example 4] ~ [Example 5]

The MSE was added to and dissolved in the under-described hydrophilic ingredients in an amount of 0.2 wt.% (Example 4) or 3.0 wt.% (Example 5). Then, the resulting solution was mixed with the under-described lipophilic ingredients at 80°C. While stirring, the resultant was cooled down to 50°C where an aromatic ingredient was added and stirring was further continued until 30°C was reached. Thus, hair creams were prepared.

| Starting ingredient | Content (wt.%) |
|---|---|
| (Hydrophilic ingredient) | |
| Methyl parahydroxybenzoate<br>Glycerin<br>Refined water | 0.2<br>5.0<br>balance |
| (Lipophilic ingredient) | |
| Liquid paraffin<br>Stearic acid<br>Cetanol<br>Sorbitan monooleate<br>Polyoxyethylene sorbitan mono-oleate (20 E.O.)<br>Isopropylmethylphenol | 30.0<br>5.0<br>5.0<br>3.0<br>3.0<br>0.1 |
| (Aromatic ingredient) | |
| Aromatic | 0.2 |

[Comparative Example 4] ~ [Comparative Example 6]

Hair creams were prepared in the same manner as Example 4, except that the MSE was replaced by 0.1 wt.% of red pepper tinctures (Comparative Example 4), 0.1 wt.% of biotin (Comparative Example 5) or 2.0 wt.% of panthenol (Comparative Example 6).

[Example 6] ~ [Example 8]

The MSE was added to and dissolved in the under-described hydrophilic ingredients in an amount of 0.1 wt.% (Example 6), 1.0 wt.% (Example 7) or 4.0 wt.% (Example 8). After dissolving, the resulting solution was admixed with the under-described lipophilic ingredients which had been homogeneously dissolved by heating and stirring at 75°C for 5 minutes. The mixture was stirred into a homogeneous dispersion which was then cooled down to 30°C while stirring. Thus, skin milks were prepared.

| Starting ingredient | Content (wt.%) |
|---|---|
| (Hydrophilic ingredient) | |
| Sodium N-lauroylglutamate | 2.0 |
| Methyl parahydroxybenzoate | 0.2 |
| Refined water | balance |
| (Lipophilic ingredient) | |
| Liquid paraffin | 20.0 |
| Stearyl alcohol | 5.0 |
| Isopropyl myristate | 1.5 |

[Comparative Example 7] ~ [Comparative Example 8]

Skin milks were prepared in the same manner as Example 6, except that the MSE was replaced by 5.0 wt.% of γ-oryzanol (Comparative Example 7) or 0.1 wt.% of ethyl nicotinate (Comparative Example 8).

[Example 9] ~ [Example 10]

The MSE was added to the under-described hydrophilic ingredients which had been dissolved by heating at 80°C, in an amount of 0.2 wt.% (Example 9) or 4.0 wt.% (Example 10). After dissolving, the resulting solution was admixed with the under-described lipophilic ingredients which had been dissolved by heating at 80°C. Then, the mixture was stirred into a homogeneous dispersion which was then cooled down to 30°C while stirring. Thus, skin creams were prepared.

| Starting ingredient | Content (wt.%) |
|---|---|
| (Hydrophilic ingredient) | |
| Glycerin | 5.0 |
| Methyl parahydroxybenzoate | 0.1 |
| Refined water | balance |
| (Lipophilic ingredient) | |
| Squalane | 10.0 |
| Olive oil | 10.0 |
| Solid paraffin | 5.0 |
| Cetanol | 4.0 |
| Sorbitan monostearate | 2.0 |
| Polyoxyethylene sorbitan monostearate (20 E.O.) | 2.0 |

[Comparative Example 9] ~ [Comparative Example 10]

Skin creams were prepared in the same manner as Example 9, except that the MSE was replaced by 5.0 wt.% of panthenol (Comparative Example 9) or 0.1 wt.% of biotin (Comparative Example 10).

17

[Example 11]

The under-described lipophilic ingredients were dissolved by heating at 80°C, and the under-described hydrophilic ingredients which had been dissolved by heating at 80°C were added thereto and mixed by stirring. Then, while stirring, the mixture was gradually cooled down to room temperature. Thus, a water-absorbing ointment blended with MSE was prepared.

| Starting ingredient | Content (wt.%) |
|---|---|
| (Hydrophilic ingredient) | |
| MSE | 5.0 |
| Methyl parahydroxybenzoate | 0.1 |
| Refined water | 31.3 |
| (Lipophilic ingredient) | |
| White petrolatum | 40.0 |
| Cetanol | 18.0 |
| Sorbitan sesquioleate | 5.0 |
| Lauromacrogol | 0.1 |
| Butylparaben | 1.5 |

[Comparative Example 11]

A water-absorbing ointment was prepared in the same manner as Example 11, except that the MSE was replaced by refined water.

[Example 12]

To 50 g of the MSE, a mixture of lactose, corn starch and a crystalline cellulose was added together with hydroxypropyl cellulose dissolved in 30 g of refined water, in the under-described amounts, and kneaded thoroughly. This kneaded mixture was passed through a 20-mesh sieve and granulated into granules followed by drying. The obtained granules were mixed with magnesium stearate and tableted into tablets of 200 mg/tablet. Thus, tablets containing 100 mg of active ingredients per tablet were obtained.

| (Ingredient) | Content (g) |
|---|---|
| Lactose | 10 |
| Corn starch | 30 |
| Crystalline cellulose | 8 |
| Hydroxypropylcellulose | 1 |
| Magnesium stearate | 1 |

[Example 13]

To 100 g of the MSE, lactose, corn starch and a crystalline cellulose were added in the under-described amounts and thoroughly mixed together. Capsules of the size No. 2 were loaded with 300 mg/capsule of the mixture. Thus, capsules containing 100 mg of active ingredients per capsule were obtained.

| (Ingredient) | Content (g) |
|---|---|
| Lactose | 100 |
| Corn starch | 50 |
| Crystalline cellulose | 47 |
| Magnesium stearate | 3 |

[Example 14]

To 100 g of the MSE, lactose and corn starch were mixed thoroughly and a hydroxypropyl cellulose dissolved in 1,000 g of refined water was added, in the under-described amounts, and kneaded thoroughly. This kneaded mixture was passed through a 20-mesh sieve and granulated. After drying, all the particle sizes were evened. Thus, granules containing 100 mg of the MSE as an active ingredient per 1 g of granules were obtained.

| (Ingredient) | Content (g) |
|---|---|
| Lactose | 470 |
| Corn starch | 400 |
| Hydroxypropylcellulose | 30 |

Industrial applicability

When the N-methyl-L-serine according to the present invention is added to a culture system of a human fibrosarcoma cell strain, the production of the procollagenase is promoted (Experimental Example-1).

In the test using animals, it has been confirmed that it is effective for medical treatments of diseases accompanied with an abnormal accumulation of collagen, for example, hepatic fibrosis (Experimental Example-2). Further, it has been confirmed that a skin collagen composition to be otherwise insolubilized with aging, is kept young (Experimental Example-6).

The N-methyl-L-serine according to the present invention is a safe compound free from toxicity and skin irritation (Experimental Examples 7 and 8).

Moreover, its effects have been recognized in the practicality tests as hair growth stimulants and skin aging inhibitors (Experimental Examples-9 and 10).

From the above, it is clearly recognized that the N-methyl-L-serine according to the present invention is efficacious, as a collagen metabolism ameliorant as well as a hair growth stimulant and a skin aging inhibitor, against collagen hypometabolism caused by aging or diseases accompanied with an abnormal accumulation of collagen.

**Claims**

1. A collagen metabolism ameliorant containing N-methyl-L-serine as an active ingredient.

2. The collagen metabolism ameliorant according to claim 1, which is efficacious against diseases accompanied with an abnormal accumulation of collagen, which diseases include hepatic and pulmonary fibroses, keloid, hypertrophic scar, scleroderma, fibrosis in the scalp and the like.

3. The collagen metabolism ameliorant according to claim 1, which is efficacious against collagen hypometabolism caused by aging.

4. A hair growth stimulant and a skin aging inhibitor, containing N-methyl-L-serine as an active ingredient.

5. The hair growth stimulant and the skin aging inhibitor according to claim 4, wherein said hair growth stimulant is a hair-restoring cosmetic and said skin aging inhibitor is a dermal cosmetic.

6. A pharmaceutical composition containing N-methyl-L-serine as an active ingredient.

7. Use of N-methyl-L-serine for preparing a pharmaceutical composition for treating diseases accompanied with an abnormal accumulation of collagen, which diseases include hepatic and pulmonary fibroses, keloid, hypertrophic scar, scleroderma, fibrosis in the scalp and the like.

8. Use of N-methyl-L-serine for preparing a pharmaceutical composition according to claim 7, which is efficacious against collagen hypometabolism caused by aging.

9. Use of a collagen metabolism ameliorant according to claim 1 as a hair growth stimulant.

10. Use of a collagen metabolism ameliorant according to claim 1 as a skin aging inhibitor.

**Patentansprüche**

1. Mittel zur Verbesserung des Collagenmetabolismus, enthaltend N-Methyl-L-serin als wirksamen Bestandteil.

2. Mittel zur Verbesserung des Collagenmetabolismus gemäß Anspruch 1, welches wirksam ist gegen Krankheiten, die mit einer anomalen Collagenakkumulation einhergehen, wobei diese Krankheiten Leber- und Lungenfibrosen, Keloid, hypertrophe Narben, Sklerodermie, Fibrosen in der Kopfhaut und dgl. einschließen.

3. Mittel zur Verbesserung des Collagenmetabolismus gemäß Anspruch 1, welches wirksam ist gegen den durch Alterung verursachten Collagenhypometabolismus.

4. Haarwachstumsstimulierendes Mittel und Hautalterungsinhibitor, enthaltend N-Methyl-L-serin als wirksamen Bestandteil.

5. Haarwachstumsstimulierendes Mittel und Hautalterungsinhibitor gemäß Anspruch 4, wobei das Haarwachstumsstimulans ein Haar-wiederherstellendes Kosmetikum ist und der Hautalterungsinhibitor ein Dermalkosmetikum ist.

6. Pharmazeutische Zusammensetzung, enthaltend N-Methyl-L-serin als Wirkstoff.

7. Verwendung von N-Methyl-L-serin zur Herstellung einer pharmazeutischen Zusammensetzung zur Behandlung von Krankheiten, die mit einer anomalen Collagenakkumulation einhergehen, wobei diese Krankheiten Leber- und Lungenfibrosen, Keloid, hypertrophe Narben, Sklerodermie, Fibrosen in der Kopfhaut und dgl. einschließen.

8. Verwendung von N-Methyl-L-serin zur Herstellung einer pharmazeutischen Zusammensetzung gemäß Anspruch 7, welche wirksam ist gegen durch Alterung verursachten Collagenhypometabolismus.

9. Verwendung eines Mittels zur Verbesserung des Collagenmetabolismus gemäß Anspruch 1 als Haarwachstumsstimulans.

10. Verwendung eines Mittels zur Verbesserung des Collagenmetabolismus gemäß Anspruch 1 als Hautalterungsinhibitor.

**Revendications**

1. Agent améliorant le métabolisme du collagène contenant, comme principe actif, de la N-méthyl-L-sérine.

2. Agent améliorant le métabolisme du collagène conforme à la revendication 1 qui est efficace contre des maladies accompagnées d'une accumulation anormale de collagène, lesquelles maladies englobent les fibroses hépatique et pulmonaire, la chéloïde, une cicatrice hypertrophique, la sclérodermie, la fibrose du cuir chevelu et des maladies similaires.

3. Agent améliorant le métabolisme du collagène conforme à la revendication 1 qui est efficace contre l'hypométabolisme du collagène dû au vieillissement.

4. Agent stimulant la croissance des cheveux et agent inhibant le vieillissement de la peau contenant, comme principe actif, de la N-méthyl-L-sérine.

5. Agent stimulant la croissance des cheveux et agent inhibant le vieillissement de la peau conforme à la revendication 4 dans lequel ledit agent stimulant de la croissance des cheveux est un produit cosmétique pour la restauration des cheveux et ledit agent inhibant le vieillissement de la peau est un produit dermo-cosmétique.

6. Composition pharmaceutique contenant, comme principe actif, de la N-méthyl-L-sérine.

7. Utilisation de la N-méthyl-L-sérine pour la préparation d'une composition pharmaceutique pour le traitement de maladies accompagnées d'une accumulation anormale de collagène, lesquelles maladies englobent les fibroses hépatique et pulmonaire, la chéloïde, une cicatrice hypertrophique, la sclérodermie, la fibrose du cuir chevelu et des maladies similaires.

8. Utilisation de la N-méthyl-L-sérine pour la préparation d'une composition pharmaceutique conforme à la revendication 7 qui est efficace contre l'hypométabolisme du collagène dû au vieillissement.

9. Utilisation d'un agent améliorant le métabolisme du collagène conforme à la revendication 1 comme agent stimulant la croissance des cheveux.

10. Utilisation d'un agent améliorant le métabolisme du collagène conforme à la revendication 1 comme agent inhibant le vieillissssment de la peau.